# EUROPEAN PATENT APPLICATION

(11) **EP 3 704 958 A1**
(43) Date of publication of application: **09.09.2020**
(21) Application number: 17930603.0
(22) Date of filing: 31.10.2017
(51) Int. Cl.: A23L 33/135, A61P 21/00, C12N 1/20

(54) **MUSCLE-BUILDING COMPOSITION**

(71) Applicant: Morinaga Milk Industry Co., Ltd., Minato-ku Tokyo 108-8384 (JP)
(72) Inventor: TODA Kazuya, Zama-shi Kanagawa 252-8583 (JP); MINAMI Junichi, Zama-shi Kanagawa 252-8583 (JP); MATSUNAGA Yutaka, Zama-shi Kanagawa 252-8583 (JP)
(74) Representative: Vossius & Partner Patentanwälte Rechtsanwälte mbB
(86) International application number: PCT/JP2017/039273
(87) International publication number: WO 2019/087280

(57) **Abstract**

To provide a composition and the like for increasing muscle mass that can effectively increase muscle mass with fewer side effects and high safety. A composition for increasing muscle mass containing Bifidobacterium and/or a culture thereof as an active ingredient; a method for increasing muscle mass in which Bifidobacterium and/or a culture thereof is administered as an active ingredient; a method for improving or treating muscle diseases or symptoms thereof, in which Bifidobacterium and/or a culture thereof is administered as an active ingredient; Bifidobacterium and/or a culture thereof for improving or treating muscle diseases or symptoms thereof; use of Bifidobacterium and/or a culture thereof for increasing muscle mass; and use of Bifidobacterium and/or a culture thereof for producing a composition for increasing muscle mass containing the Bifidobacterium and/or a culture of thereof as an active ingredient.

## Description

### [Technical Field]

The present technology relates to a composition for increasing muscle mass, a pharmaceutical composition for increasing muscle mass, and a food and beverage composition for increasing muscle mass containing Bifidobacterium and/or a culture thereof as active ingredient.

### [Background Art]

Since muscles are the basis for everyday life, such as standing, walking, and maintaining posture, everyone, regardless of age or gender, has a desire to maintain as much muscle mass as possible to maintain quality of life, and to increase their muscle mass, if possible. In addition, persons who like to actively exercise and athletes (for example, athletes, sports enthusiasts, competitors, and the like) have a desire to increase as much of their muscle as possible to improve performance.

Furthermore, as health trends are strengthening, how to increase muscle mass is a challenge for improving quality of life (abbreviated as "QOL"), improving frailty (an intermediate state between a healthy state and a care-required state, in which nursing care support is required in daily life), and as countermeasures in locomotive syndrome (abbreviated as "locomo").

Muscles typically account for about 40% of body weight, 70 to 80% of which is water, and most of the rest is composed of protein and free amino acids. Therefore, proteins or amino acids are also important as nutrients for increasing or maintaining muscle because of their composition. For amino acids, branched amino acids (especially leucine) have been shown to serve as stimulants to promote protein synthesis as well as as building blocks for protein synthesis. Therefore, supplements containing various proteins and amino acids are used to increase muscle mass (non-patent literature 1).

However, the mechanism of action of muscle mass increase is also complexly related to nutrients and protein synthesis systems, so many points are still unclear. Therefore, since there are various approaches to increasing muscle mass, further searches for substances capable of increasing muscle mass are being diligently carried out.

### [Prior Art Literature]

### [Non-patent Literature]

[Non-patent Literature 1] Written by Yoshiharu Shimomura, Sports and Health Nutrition (Third Edition), December 15, 2010, First Printing of the Third Edition

### [Summary of the Invention]

### [Problems to be Solved by the Invention]

With the foregoing circumstances in view, a substance that has fewer side effects, is safe, and can effectively increase muscle mass is desirable because cases in which it will be ingested continuously over a long time to obtain the effect of increased muscle mass is also conceivable. A main object of the present technology is to provide a composition for muscle mass increase capable of effectively increasing muscle mass with fewer side effects and high safety.

### [Solution Means]

As a result of diligent research carried out by the present inventors, Bifidobacterium and/or a culture of thereof have an effect of myotube hypertrophy and also have an effect of increasing muscle mass in subject tests; thereby the present invention has been completed.

The present invention has the following configurations:
[1] A composition for increasing muscle mass containing Bifidobacterium and/or a culture thereof as an active ingredient.
[2] A method for increasing muscle mass in which Bifidobacterium and/or a culture thereof is administered as an active ingredient.
[3] A method for improving or treating muscle diseases or symptoms thereof, in which Bifidobacterium and/or a culture thereof is administered as an active ingredient.
[4] Bifidobacterium and/or a culture thereof for increasing muscle mass.
[5] Bifidobacterium and/or a culture thereof for improving or treating muscle diseases or symptoms thereof.
[6] Use of Bifidobacterium and/or a culture thereof for increasing muscle mass.
[7] Use of Bifidobacterium and/or a culture thereof for producing a composition for increasing muscle mass containing the Bifidobacterium and/or the culture thereof as an active ingredient.
[8] The composition may be a pharmaceutical composition or a food and beverage composition.

### [Effect of the Invention]

According to the present invention, it is possible to provide a composition that is able to increase muscle mass by effectively increasing muscle mass with fewer side effects and high safety.

It should be noted that the effects described herein are not necessarily limited and may be any of the effects described in the present technology.

### [Brief Description of the Drawings]

FIG. 1 is a photograph of the gastrocnemius muscle of a mouse (Bifidobacterium-administered group) in test example 3 observed under a microscope. Reference numeral 1 in FIG. 1 indicates a myotube.
FIG. 2 is a photograph of the gastrocnemius muscle of a mouse (control group) in test example 3 observed under a microscope. Reference numeral 1 in FIG. 2 indicates a myotube, and reference numeral 2 indicates connective tissue.

### [Embodiment]

Next, a preferable embodiment of the present invention will be described in detail. However, the present invention is not limited to the following preferable embodiment and can be freely modified within the scope of the present invention. It should be noted that percentages in the present specification are displayed by mass unless otherwise noted.

A composition for increasing muscle mass of the present technology has a muscle hypertrophy effect or muscle mass increasing effect, and contains a Bifidobacterium and/or a culture thereof as an active ingredient.

It should be noted that "muscle hypertrophy" in the present specification refers to the increase in muscle volume (size) by thickening of myotubes or myofibers. "Muscle mass increase" in the present specification refers to increased muscle volume and/or increased muscle weight.

### <Bifidobacterium genus bacteria>

Bifidobacterium used in the present technology are not particularly limited unless they impair the effects of the present invention, and known Bifidobacterium may be used. For Bifidobacterium used in the present technology, those that have the ability to produce a substance expressing a muscle hypertrophy effect or a substance (also including bacterial cell components) expressing a muscle mass increase effect are suitable.

Examples of such Bifidobacterium include Bifidobacterium breve, Bifidobacterium longum subsp. longum, Bifidobacterium bifidum, Bifidobacterium adolescentis, Bifidobacterium angulatum, Bifidobacterium dentium, Bifidobacterium pseudocatenulatum, Bifidobacterium animalis subsp. lactis, Bifidobacterium pseudolongum subsp. globosum, Bifidobacterium pseudolongum subsp. pseudolongum, Bifidobacterium thermophilum, and the like. It should be noted that Bifidobacterium longum subsp. longum may simply be described as Bifidobacterium longum.

Furthermore, among the foregoing Bifidobacterium, any of Bifidobacterium breve, Bifidobacterium longum subsp. longum, Bifidobacterium bifidum, Bifidobacterium adolescentis, Bifidobacterium dentium, Bifidobacterium animalis subsp. lactis, Bifidobacterium pseudolongum subsp. globosum, Bifidobacterium pseudolongum subsp. pseudolongum, or Bifidobacterium thermophilum are more preferable.

Furthermore, among the above Bifidobacterium, Bifidobacterium breve FERM BP-11175, Bifidobacterium breve ATCC 15700, Bifidobacterium longum subsp. longum ATCC 15707, Bifidobacterium bifidum ATCC 29521, Bifidobacterium adolescentis ATCC 15703, Bifidobacterium dentifium DSM 20436, Bifidobacterium animalis subsp. lactis DSM 10140, Bifidobacterium pseudolongum subsp. globosum JCM5820, Bifidobacterium pseudolongum subsp. pseudolongum ATCC 25526, or Bifidobacterium thermophilum ATCC 25525 are even more preferable.

In addition, for Bifidobacterium used in the present technology, one type or two types may be selected from the above group, and only one type or any two or more types may be used.

It should be noted that the bacteria with ATCC numbers are known bacteria that can be obtained from the American Type Culture Collection (address: 12301 Parklawn Drive, Rockville, Maryland 20852, United States of America). For example, ATCC 15700 (ATCC^{(R)}15700™), ATCC 15707 (ATCC^{(R)}15707™), ATCC 29521 (ATCC^{(R)}29521™), ATCC 15703 (ATCC^{(R)}15703™), ATCC 27535 (ATCC^{(R)}27535™), ATCC 27919 (ATCC^{(R)}27919™), ATCC 25526 (ATCC^{(R)}25526™), ATCC 25525 (ATCC^{(R)}25525™), and the like can be obtained (https://www.atcc.org/products/all/,[online]: searched on October 17, 2017).

Also, bacteria with DSM numbers are known bacteria that can be obtained from the DSMZ (German Collection of Microorganisms and Cell Cultures) (Inhoffenstr. 7B, D38124 Braunschweig, GERMANY). For example, DSM20436 (DSM No: 20436), DSM10140 (DSM No: 10140), and the like can be obtained (https://www.dsmz.de/catalogues/catalogue-microorganisms.html, [online]: searched on October 17, 2017).

In addition, bacteria with JCM numbers are known bacteria that can be obtained from JCM (address: 3-1-1 Koyadai, Tsukuba-shi, Ibaraki-ken, 3050074 RIKEN, Institute of Physical and Chemical Research, Bio Resource Center, Microbial Materials Development Office). For example, JCM5820 (JCM 5820^{T}, http://www.jcm.riken.jp/cgi-bin/jcm/jcm_number?JCM=5820,[online]: searched on October 17, 2017) and the like can be obtained.

Bacteria with the accession number of FERM BP-11175 were internationally deposited based on the Budapest Treaty at the National Institute of Advanced Industrial Science and Technology International Patent Organism Deposit Center (currently: National Institute for Product Evaluation Technology (NITE) International Patent Organism Deposit Center (IPOD) (NITE-IPOD) (postcode: 292-0818, address: 2-5-8-120, Kazusakamatari, Kisarazu-shi, Chiba-ken) on August 25, 2009.

The Bifidobacterium used as described herein may be a mutant strain of the Bifidobacterium, as long as they have properties that can satisfy the objects of the present invention (for example, muscle mass increase effect). Bifidobacterium are anaerobic bacteria, so they can be grown under anaerobic conditions, and it is believed that they cannot grow in air. Also, the mutant strain preferably has the same bacteriological properties as the foregoing Bifidobacterium and has a muscle mass increase promoting effect equivalent to or greater than that of the foregoing Bifidobacterium. Whether a mutant strain has a "muscle mass increase promoting effect equivalent to or greater than that of' Bifidobacterium described above can be confirmed, for example, by the method of the test example described below.

Such a mutant strain may be constructed by non-artificially introducing a mutation to the above Bifidobacterium. In addition, it may be constructed by introducing a mutation into the aforementioned bacteria through treatment using a mutagen such as UV, and a mutation may be introduced into the aforementioned strains using various genetic manipulation methods.

The Bifidobacterium and/or the culture thereof used in the present technology can be readily obtained by culturing Bifidobacterium by normal methods.

It should be noted that the Bifidobacterium and/or the culture thereof also includes products produced by the bacteria (for example, bacterial cell components, metabolites, and the like), and such products include separated and purified products (for example, supernatants, extracts, and the like) from the culture thereof and/or bacterial processed products (for example, bacterial cell components such as peptidoglycans and cell walls, and the like).

The method for culturing the bacteria is not particularly limited as long as Bifidobacterium can proliferate, and culture can be carried out under appropriate conditions according to the properties thereof. For example, the culturing temperature may be 25 to 50°C, preferably 35 to 42°C. In addition, the culture is also preferably carried out under anaerobic conditions; for example, it can be cultured while aerating the anaerobic gas such as carbon dioxide gas. In addition, it may also be cultured under microaerobic conditions such as liquid static culturing.

The medium for culturing Bifidobacterium used in the present technology is not particularly limited, and media commonly used for culturing Bifidobacterium may be used. That is, as a carbon source, for example, sugars such as glucose, galactose, lactose, arabinose, mannose, sucrose, starch, starch hydrolysate, and waste molasses may be used according to assimilation. As a nitrogen source, for example, ammonium salts such as ammonia, ammonium sulfate, ammonium chloride, and ammonium nitrate, and nitrate salts may be used. In addition, as inorganic salts, for example, sodium chloride, potassium chloride, potassium phosphate, magnesium sulfate, calcium chloride, calcium nitrate, manganese chloride, ferrous sulfate, and the like may be used. In addition, organic components such as peptone, soybean powder, defatted soybean cake, meat extract, and yeast extract may also be used.

The Bifidobacterium used in the present technology can be used in a form of bacteria themselves, cultures or dried products thereof, separated and purified products from the cultures thereof (for example, culture supernatant), or bacterial processed products thereof.

That is, the Bifidobacterium or cultures obtained by culturing the Bifidobacterium may be used as-is, only the culture supernatant may be separated from the culture and used, the culture or culture supernatant may be diluted or concentrated and used, or the bacteria collected from the culture may be used. In addition, the product thereof may also be collected by separation and purification from the culture. In addition, the culture or culture supernatant may also be dried. In addition, the Bifidobacterium used in the present invention may be viable or dead bacteria and may include both viable and dead bacteria.

In addition, separation and purification from the culture can be carried out by known separation and purification methods, such as centrifugation, salting out, solvent extraction (polar solvents, non-polar solvents, or mixtures of these solvents (for example, water, ethanol, hexane, and the like)), gel filtration separation, HPLC separation, and filtration membrane separation of product having muscle mass increase effect.

In addition, the bacterial processed products thereof include, for example, cell debris, where the cell wall and cell membrane of thereof are partially or completely crushed by common methods such as ultrasonic processing and homogenization, and the like. Furthermore, the cell debris may be all fractions or a portion of fractions after the crushing, and the centrifuged supernatant after the crushing, fraction partially purified from the supernatant by ammonium sulfate treatment or the like, or a concentrate of the supernatant may be used.

The Bifidobacterium and/or the culture thereof of the present technology has a myotube or myofiber thickness increasing effect, muscle hypertrophy effect, or muscle mass increasing effect as shown in [Examples] described later.

In general, it is said that myotubes or myofibers are thickened by promoting the synthesis of muscle proteins. It is also said that thicker myotubes or myofibers increase muscle volume (size) or muscle weight.

For this reason, the Bifidobacterium and/or the culture thereof of the present technology can also be used for symptoms or diseases that can be prevented, improved, or treated by increased muscle mass (for example, muscle disease or symptoms thereof such as muscle wasting or muscle degeneration). Examples of the muscle diseases in the present specification include atony, muscular atrophy, muscular dystrophy, muscle degeneration, sarcopenia, and the like. In the present specification, muscle disease is caused by genetic factors, acquired factors, aging, and the like, and muscle wasting is characterized by progressive loss of muscle mass, weakening, and regression of muscles.

In addition, the Bifidobacterium and/or the culture thereof of the present technology has a myotube or myofiber thickness increasing effect, muscle hypertrophy effect, or muscle mass increasing effect, and the type of muscle (fast muscle and/or slow muscle) is not limited, but the mass increasing effect is more likely expressed on muscles that work hard during sports.

In addition, the Bifidobacterium and/or the culture thereof of the present technology can increase muscle mass even in the normal life, and muscle mass can also be increased by exercise.

In addition, the Bifidobacterium and/or the culture thereof used in the present technology can be continuously ingested over a long period of time since the bacteria has few side effects and high safety.

The present technique can also be used for a purpose of enhancing muscle mass with fewer side effects by promoting muscle protein synthesis and muscle hypertrophy. The present technology can also be effectively used for increasing muscle strengthening effects by training; prevention or improvement of sarcopenia, frailty, locomotive syndrome; and prevention, improvement, or treatment of muscle atrophy-related diseases or symptoms.

Therefore, the Bifidobacterium and/or the culture thereof of the present technology can be included in the composition for increasing muscle mass as an active ingredient, and because it is highly safe, it can be used in a wide variety of applications such as pharmaceuticals, food and beverages, cosmetics, and feed. These products may be produced by known production methods suitable for each application by appropriately using optional components suitable for each application.

The Bifidobacterium and/or the culture thereof of the present technology itself can be used as-is, or may be combined with a normal carrier, diluent, or the like, which is acceptable in terms of physiology, medicine, or food and beverage.

In addition, the Bifidobacterium and/or the culture thereof of the present technology can be used for the production of various formulations, compositions, or the like. In addition, the present technology can also be used as the Bifidobacterium and/or the culture thereof for increasing muscle mass.

Administration or ingestion of the Bifidobacterium and/or the culture thereof of the present technology is preferably continued for at least 4 weeks, more preferably for at least 8 weeks, and desirably continued daily.

The amount of the Bifidobacterium used in the present technology is not particularly limited because it is highly safe, but for example, 1 x 10⁶ to 1 x 10¹²CFU/kg body weight/day is preferable, 1 x 10⁷ to 1 x 10¹¹CFU/kg body weight/day is more preferable, and 1 x 10⁸ to 1 x 10¹⁰ CFU/kg body weight/day is still more preferable. Alternatively, as the amount (dose) used per individual (Body weight), 10⁷ to 10¹⁴CFU/day is preferable, 10⁸ to 10¹³ CFU/day is more preferable, and 10⁹ to 10¹² CFU/day is still more preferable.

In addition, for the amount used of the culture of the Bifidobacterium or the separated and purified products derived therefrom (for example, culture supernatant), 0. 01 to 100 mL/kg body weight/day is preferable, and 0. 1 to 10 mL/kg body weight/day is more preferable. It should be noted that, in the present technology, CFU refers to colony forming units. If the bacterium is a dead bacterium, CFU can be replaced with individual cells.

The Bifidobacterium and/or the culture thereof of the present technology may be used in humans, which is a subject, or non-human animals (preferably mammals), humans and pets are preferable, and humans are more preferable.

The subject is not particularly limited if the individual wishes to increase muscle mass, and examples thereof include infants, children, adults, healthy individuals, athletes, middle age, elderly, those who have symptoms of muscle disease, and the like.

In addition, the present technology may be for therapeutic use or non-therapeutic use. "Non-therapeutic purpose" is a concept that does not include medical actions, that is, treatment action of the human body by medical treatment. For example, health promotion, cosmetic activities, and the like are included.

"Improvement" refers to betterment of a disease, symptom, or condition; prevention and delay of worsening of a disease, symptom, or condition; reversal, prevention, or delay of progression of the disease or symptom.

"Prevention" refers to the preclusion or delay of the onset of a disease or symptom in the subject, or a reduction in the risk of a disease or symptom in the application subject.

### <Pharmaceutical compositions>

A composition for increasing muscle mass of the present technology can be used as a pharmaceutical composition. The pharmaceutical composition of the present technology can be used to prevent, improve, and/or treat muscle diseases, symptoms thereof, muscle atrophy-related diseases, or symptoms thereof. The present technology is particularly suitable for the purpose of increasing muscle mass. As muscle atrophy-related diseases or symptoms thereof, disuse muscular atrophy associated with sarcopenia, being bedridden, lack of exercise, zero gravity flight, and extremity fixation in the treatment of injuries; neurogenic muscular atrophy such as amyotrophic lateral sclerosis (ALS), spinal progressive muscular atrophy, acute spinal keratitis (polio), Guillain-Barre syndrome; and myogenic muscular atrophy such as muscular dystrophy can be exemplified.

In addition, the pharmaceutical compositions of the present technology can be administered without concern to patients suffering from a variety of diseases, since the active ingredients include the Bifidobacterium and/or the culture thereof (for example, culture supernatant of the culture thereof and/or bacterial processed products thereof) that have been used for many years as oral composition components. In addition, since Bifidobacterium are present in the intestines of animals, it is expected that the present technology will not cause side effects even if administered continuously over a long period of time. In addition, the Bifidobacterium and/or the culture thereof can be safely administered to infants and children as well. Therefore, the present technology is also suitable for prevention, improvement, and/or treatment of disease or symptoms thereof in infants and children.

When the composition for increasing muscle mass according to the present technology is utilized as a pharmaceutical composition, the pharmaceutical composition may be either oral or parenteral, and may be formulated into the desired dosage form as appropriate, according to the administration method. For example, in the case of oral administration, it may be formulated into solid formulations such as powders, granules, tablets, and capsules; and liquid formulations such as solutions, syrups, suspensions, and emulsions. In addition, in the case of parenteral administration, it may be formulated into a suppository, ointment, and the like.

In addition, when formulating, ingredients such as excipients, pH adjusting agents, colorants, and flavoring agents that are normally used for formulation may be used for the pharmaceutical compositions according to the present technology. In addition, as long as the effect of the present invention is not impaired, for the pharmaceutical composition according to the present technology, ingredients having effects of prevention, improvement, and/or treatment on known or future muscular diseases, muscular atrophy-related diseases, or their symptoms may also be used.

In addition, formulation can be carried out by a method that is appropriate and well known according to the dosage form. When formulating, formulation may be carried out by blending a formulation carrier as appropriate.

The ingestion amount or administered amount of the pharmaceutical composition of the present technology may be selected as appropriate according to the dosage form, but for example, the intake or administered amount of Bifidobacterium per day per kg of body weight is preferably 1 x 10⁶ to 1 x 10¹²CFU/kg body weight/day, more preferably 1 x 10⁷ to 1 x 10¹¹CFU/kg body weight/day, and still more preferably 1 x 10⁸ to 1 x 10¹⁰CFU/kg body weight/day. Alternatively, the intake or administered amount per individual (Body weight) is preferably 10⁷ to 10¹⁴CFU/day, more preferably 10⁸ to 10¹³ CFU/day, and still more preferably 10⁹ to 10¹²CFU/day.

In addition, when using the culture of the Bifidobacterium, the separated and purified products derived from the bacteria (for example, culture supernatant), or the bacterial processed products thereof, the intake or administered amount is preferably the intake or administered amount when converted to the intake or administered amount of the Bifidobacterium.

In addition, when using the culture of the Bifidobacterium or the separated and purified products derived from the culture thereof (for example, culture supernatant), as the administered amount for a daily dose per kg of body weight, 0.01 to 100 mL is preferable, and 0.1 to 10 mL is more preferable. At this time, as the separated and purified product (for example, culture supernatant) thereof, using a known medium, a separated and purified product (for example, supernatant) obtained from a culture cultured so that the number of the Bifidobacterium will be 1 x 10⁷ to 1 x 10¹¹CFU/mL is preferable, and a separated and purified product (for example, culture supernatant) obtained from a culture cultured so that it will be 1 x 10⁸ to 1 x 10¹⁰CFU/mL is more preferable.

Furthermore, the pharmaceutical composition of the present technology is preferably continuously ingested at the intake or administered amount for at least 4 weeks every day, is more preferably continuously ingested for at least 8 weeks every day, and is preferably continuously ingested for at least 12 weeks every day.

In addition, the concentration of the Bifidobacterium in the pharmaceutical composition of the present technology may be selected as appropriate based on the intake or administered amount. For example, it may be 1 x 10⁶ to 1 x 10¹²CFU/g or 1 x 10⁶ to 1 x 10¹²CFU/mL, preferably 1 x 10⁷ to 1 x 10¹¹CFU/g or 1 x 10⁷ to 1 x 10¹¹CFU/mL, and more preferably 1 x 10⁸ to 1 x 10¹⁰CFU/g or 1 x 10⁸ to 1 x 10¹⁰CFU/mL. If the bacterium is a dead bacterium, CFU can be replaced with individual cells.

Also, when using the culture of Bifidobacterium, the separated and purified products derived from the culture thereof (for example, culture supernatant), or the bacterial processed product thereof, the foregoing concentration is preferably the concentration is preferably the foregoing content when converted to the content of Bifidobacterium . Furthermore, when the culture of the Bifidobacterium or the separated and purified products derived from the culture thereof (for example, culture supernatant) is used, 0.01 to 100 mL thereof is preferably present, and 0.1 to 10 mL thereof is preferably present.

Also, as the formulation carrier, various organic or inorganic carriers can be used according to the dosage form. Carriers in the case of solid formulations include, for example, excipients, binders, disintegrants, lubricants, stabilizers, orthodontic fresheners, and the like.

Examples of excipients include sugar derivatives such as lactose, sucrose, glucose, mannitol, and sorbitol; starch derivatives such as corn starch, potato starch, α-starch, dextrin, and carboxymethyl starch; cellulose derivatives such as crystalline cellulose, hydroxypropyl cellulose, hydroxypropyl methyl cellulose, carboxymethyl cellulose, and carboxymethyl cellulose calcium; gum arabic; dextran; pullulan; silicate derivatives such as light anhydrous silicic acid, synthetic aluminum silicate, and magnesium metasilicate aluminate; phosphate derivatives such as calcium phosphate; carbonate derivatives such as calcium carbonate; sulfate derivatives such as calcium sulfate; and the like.

Binders include, for example, the above excipients, as well as gelatin; polyvinyl pyrrolidone; macrogol; and the like.

Disintegrants include, for example, the above excipients, as well as chemically modified starches or cellulose derivatives such as croscarmellose sodium, sodium starch glycolate, and crosslinked polyvinyl pyrrolidone.

Examples of lubricants include talc; stearic acid; stearic acid metal salts such as calcium stearate and magnesium stearate; colloidal silica; waxes such as pea gum and spermaceti; boric acid; glycol; carboxylic acids such as fumaric acid and adipic acid; sodium carboxylates such as sodium benzoate; sulfates such as sodium sulfate; leucine; lauryl sulfates such as sodium lauryl sulfate and magnesium lauryl sulfate; silicates such as anhydrous silicic acid and silicic acid hydrate; starch derivatives; and the like.

Stabilizers include, for example, paraoxybenzoate esters such as methyl paraben and propyl paraben; alcohols such as chlorobutanol, benzyl alcohol, and phenylethyl alcohol; benzalkonium chloride; acetic anhydride; sorbic acid; and the like.

Flavoring agents include, for example, sweeteners, acidulants, fragrances, and the like. It should be noted that the carriers used in the case of liquid for oral administration include solvents such as water, flavoring agents, and the like.

### <Food and beverage composition>

In addition, the composition for increasing muscle mass of the present technology can be used as a food and beverage composition. The food and beverage composition of the present technology may be produced by adding the Bifidobacterium and/or the culture thereof (for example, culture supernatant of the culture thereof and/or bacterial processed products thereof) to the known food and beverage, or by mixing the Bifidobacterium and/or the culture thereof (for example, culture supernatant of the culture of thereof and/or bacterial processed products of thereof) into the food and beverage raw materials to produce a new food and beverage composition.

The food and beverage composition in the present technology is not limited to a form such as liquid, paste-like, solid, and powder forms, and such examples include not only tablet confectioneries, liquid foods, feeds (including those for pets), and the like, but also includes wheat flour products, instant foods, processed agricultural products, processed fishery products, processed livestock products, milk and dairy products such as fermented milk or cheese, fats and oils, basic seasonings, compound seasonings and foods, frozen foods, confectioneries, beverages, other commercial products, and the like.

In addition, for the food and beverage composition in the present technology, as long as the effect of the present invention is not impaired, a component having an effect of increasing muscle mass which is well known or known in the future or a component supporting the effect of increasing muscle mass may be used. For example, the food and beverage compositions in the present technology may be prepared by combining the Bifidobacterium and/or the culture thereof with a variety of proteins or mixtures or degraded products thereof, such as whey protein, casein protein, soy protein, or pea protein; amino acids such as leucine, valine, isoleucine, or glutamine; vitamins such as vitamins B6 or vitamin C; creatine; citric acid; or components such as fish oil.

In addition, the food and beverage composition defined in the present technology can be provided and sold as a food and beverage product in which use (including health use) such as prevention of muscle disease or a muscle atrophy-related disease or symptoms thereof, reduction of the risk of disease or symptoms, relief of symptoms of disease, and/or treatment of disease or symptoms thereof is labeled. In addition, it can be provided and sold by labeling the ingestion subject of the food and beverage, such as "athletes," "those who want to increase muscle," "those who are concerned about a decline in muscle strength associated with lack of exercise," "those who are concerned about muscle weakness due to aging," "those who are concerned about a decline in muscle strength with age," "those who want to improve frailty," and the like.

"Labeling" acts include all actions to inform the use to the user, and any expression that the use may be envisioned or inferred, regardless of the purpose of the label, the content of the label, the subject matter, media, or the like to be labeled, all fall under the "labeling" acts of the present invention.

In addition, it is also preferable that the "labeling" is carried out by an expression that allows the user to directly recognize the use. Specific examples include the act that a product or the packaging related to the food and beverage product on which use is described is transferred, delivered, labeled for transferring or delivering, or imported, the act that the use is described on advertisements on the products, price lists, or transaction documents, and they are labeled, distributed, or provided with a description of the information including these contents and the use by an electromagnetic (internet or the like) method, and the like.

On the other hand, it is preferable that the content of the label is a label approved by a government or the like (for example, a label and the like that are approved based on the various systems prescribed by a government, and that is carried out in accordance with such approval). In addition, it is preferable to attach such label contents to advertising materials on the sales sites such as packaging, containers, catalogs, pamphlets, and POP, other documents, and the like.

In addition, "labeling" also includes labeling as health foods, functional foods, enteral nutrition foods, special purpose foods, health functional foods, specified health foods, nutritional functional foods, functional labeling foods, and quasi-drugs. Among them, in particular, labels approved by the Consumer Affairs Agency, for example, a label approved in systems related to specified health foods, nutritional functional foods, or functional labeling foods, or similar systems, and the like are included. Specifically, labeling as a specified health food, labeling as a conditionally specified health food, labeling that it affects the structure and function of the body, labeling that it reduces the risk of disease, and labeling of functionality based on scientific evidence, and the like are included, and more specifically, labeling as a specified health food designated in Cabinet Office Ordinance concerning permission for special purpose labeling stipulated in the Health Promotion Act (Cabinet Office Ordinance No. 57, August 31, 2009) (especially the labeling of the use for health) and similar labeling are typical examples.

Intake of the food and beverage composition of the present technology may be selected as appropriate, but for example, the intake of Bifidobacterium per day per kg of body weight is preferably 1 x 10⁶ to 1 x 10¹²CFU/kg body weight/day, more preferably 1 x 10⁷ to 1 x 10¹¹CFU/kg body weight/day, and still more preferably, 1 x 10⁸ to 1 x 10¹⁰CFU/kg body weight/day. Alternatively, the intake or administered amount per individual (body weight) is preferably 10⁷ to 10¹⁴CFU/day, more preferably 10⁸ to 10¹³CFU/day, and still more preferably 10⁹ to 10¹²CFU/day. If the bacterium is a dead bacterium, CFU can be replaced with individual cells.

Also, when using the culture of Bifidobacterium or the bacterial processed products of thereof, the intake is preferably intake when converted to the intake of Bifidobacterium.

In addition, when using the culture of Bifidobacterium or the separated and purified products derived from the culture (for example, culture supernatant), as the intake or administered amount per kg of body weight per day, 0. 01 to 100 mL is preferable, and 0. 1 to 10 mL is more preferable. At this time, as the separated and purified product (for example, culture supernatant) derived from the culture thereof, using a known medium, a separated and purified product (for example, supernatant) obtained from a culture cultured so that the concentration of the Bifidobacterium will be 1 x 10⁷ to 1 x 10¹¹CFU/mL is preferable, and a separated and purified product (for example, culture supernatant) obtained from a culture cultured so that it will be 1 x 10⁸ to 1 x 10¹⁰CFU/mL is more preferable.

Furthermore, the food and beverage composition of the present technology is preferably continuously ingested at the intake or administered amount for at least 4 weeks every day, is more preferably continuously ingested for at least 8 weeks every day, and is preferably continuously ingested for at least 12 weeks every day.

In addition, the content of the Bifidobacterium in the food and beverage composition of the present technology may be selected as appropriate based on the intake, and for example, it may be 1 x 10⁶ to 1 x 10¹²CFU/g or 1 x 10⁶ to 1 x 10¹²CFU/mL, preferably 1 x 10⁷ to 1 x 10¹¹CFU/g or 1 x 10⁷ to 1 x 10¹¹CFU/mL, more preferably 1 x 10⁸ to 1 x 10¹⁰CFU/g or 1 x 10⁸ to 1 x 10¹⁰CFU/mL. If the bacterium is a dead bacterium, CFU can be replaced with individual cells. Also, when using the culture of Bifidobacterium or the bacterial processed product thereof, the concentration is preferably the foregoing content when converted to the content of Bifidobacterium. Furthermore, when the separated and purified product derived from the culture of the Bifidobacterium (for example, culture supernatant) is used, 0. 01 to 100 mL thereof is preferably present, and 0. 1 to 10 mL thereof is more preferably present.

The present technology can also adopt the following configurations:
[1] A composition for increasing muscle mass containing the Bifidobacterium and/or the culture thereof as an active ingredient. The composition is suitable for oral ingestion. Pharmaceutical compositions or food and beverage compositions are suitable for the compositions.
[2] A method for increasing muscle mass in which Bifidobacterium and/or a culture of thereof is administered as an active ingredient. The administration is suitable for oral ingestion.
[3] A method for improving or treating muscle diseases or symptoms thereof, in which Bifidobacterium and/or a culture of thereof is administered as an active ingredient. The administration is suitable for oral ingestion. The muscle disease or symptoms thereof are suitable as muscle atrophy-related diseases or symptoms thereof.
[4] Bifidobacterium and/or a culture thereof for increasing muscle mass, or use thereof.
[5] Bifidobacterium and/or a culture thereof for improving or treating muscle diseases or symptoms thereof, or use thereof.
[6] Use of Bifidobacterium and/or a culture thereof for producing a composition for increasing muscle mass containing the Bifidobacterium and/or the culture thereof as an active ingredient. The composition is suitable for oral ingestion. Pharmaceutical compositions or food and beverage compositions are suitable for the compositions.
[7] A method for producing a food and beverage composition for increasing muscle mass, wherein the food and beverage composition contains Bifidobacterium and/or a culture thereof as an active ingredient. The food and beverage composition is suitably a nutritious food or a fermented food or beverage (for example, fermented milk).
[8] The composition, bacteria, or culture, use, or method thereof according to any one of [1] to [7], wherein the Bifidobacterium is/are a species or a plurality of species selected from the group consisting of Bifidobacterium breve, Bifidobacterium longum subsp. longum, Bifidobacterium bifidum, Bifidobacterium adolescentis, Bifidobacterium dentium, Bifidobacterium animalis subsp. lactis, Bifidobacterium pseudolongum subsp. globosum, Bifidobacterium pseudolongum subsp. pseudolongum, and Bifidobacterium thermophilum.
[9] The composition, bacteria, or culture, use, or method thereof according to any one of [1] to [7], wherein the Bifidobacterium is/are a species or a plurality of species selected from the group consisting of Bifidobacterium breve FERM BP-11175, Bifidobacterium breve ATCC 15700, Bifidobacterium longum subsp. longum ATCC 15707, Bifidobacterium bifidum ATCC 29521, Bifidobacterium adolescentis ATCC 15703, Bifidobacterium dentifium DSM 20436, Bifidobacterium animalis subsp. lactis DSM 10140, Bifidobacterium pseudolongum subsp. globosum JCM5820, Bifidobacterium pseudolongum subsp. pseudolongum ATCC 25526, and Bifidobacterium thermophilum ATCC 25525.
[10] The composition, bacteria, or culture, use, or method of thereof according to any one of [1] to [9], wherein the administration or ingestion period is at least 4 weeks or more.
[11] The composition, bacteria, or culture, use, or method of thereof according to any one of [1] to [10], wherein the used amount of the Bifidobacterium is 1 x 10⁶ to 1 x 10¹²CFU/kg body weight/day, or the used amount of the culture is 0. 01 to 100 mL/kg body weight/day.
[12] The composition, bacteria, or culture, use, or method of thereof according to any one of [1] to [11], wherein the culture is a separated and purified product (preferably, culture supernatant).

### [Example]

The present invention is described in greater detail below referring to examples, but the present invention is not limited to these examples.

### [Test Example 1]

### (1) Preparation of Bifidobacterium genus bacterial culture

90 µL of a bacterial solution of Bifidobacterium breve FERM BP-11175 (accession number: FERM BP-11175: available from NITE-IPOD), which was cryopreserved in an aqueous solution containing 10% nonfat dry milk, added to 3 mL of MRS liquid medium, and anaerobically cultured at 37°C for 16 hours. MRS liquid medium was prepared by dissolving 5.5 g of Difco Lactobacilli MRS Broth (manufactured by BD), and 50 mg of L-cysteine monohydrochloride and monohydrate (manufactured by Wako Pure Chemical Industries, Ltd.) in pure water to obtain 100 mL, adjusting pH to 6.5 with an aqueous HCl solution, and sterilizing at 121°C for 15 minutes. Next, each culture was centrifuged for 10 minutes under conditions of 4°C and 8000 x g, and then the culture supernatant was collected. The pH of the obtained culture supernatant obtained was adjusted to be within a range of pH 7. 0 ± 0. 05 with sodium hydroxide.

### (2) Cell culture test

C2C12 cells were inoculated in a 6-well plate to have 5 x 10⁴ cells/cm² and cultured for 24 hours in DMEM medium containing 10% fetal bovine serum and 1% penicillin and streptomycin. Thereafter, the culture supernatant of Bifidobacterium breve FERM BP-11175 prepared in (1) was added to the DMEM medium containing 2% Horse Serum and 1% penicillin and streptomycin. Using the sample cultured without adding the culture supernatant as a control, the medium was replaced with fresh medium every 2 days, and cultured for 7 days. After that, hematoxylin-eosin staining (HE staining) was carried out, and the thickness of 100 myotubes was randomly measured under a microscope, and the statistically significant difference was analyzed by a Dunnett's test.

### (3) Results

As a result, the diameter (mean value) of myotube cells in each sample was as shown in Table 1, and in the system into which the culture supernatant of Bifidobacterium breve FERM BP-11175 was added, the diameter of myotube cells was confirmed to increase by more than 3 µm compared to the control, and muscle mass significantly increased.

**[Table 1]**

| Table 1: Cell culture test 1 | Myotube diameter (µm) [Mean value ± SD] |
|---|---|
| Control | 16.95±6.75 |
| Bifidobacterium breve FERM BP-11175 | 19.89±5.52* |
| *P<0.05: VS control | |

### [Test Example 2]

In the same procedure as in test example 1, after preparing culture supernatants for 9 species of Bifidobacterium shown in Table 2 below, a cell culture test was carried out. Using the sample cultured without adding the culture supernatant as a control, the thickness of 250 myotubes was randomly measured under a microscope, and the statistically significant difference was analyzed by a Dunnett's test.

The following Bifidobacterium are publicly known bacteria available from ATCC, DSM, and JCM organizations.
- Bifidobacterium longum subsp. longum ATCC15707
- Bifidobacterium breve ATCC15700
- Bifidobacterium bifidum ATCC29521
- Bifidobacterium adolescentis ATCC15703
- Bifidobacterium dentium DSM20436
- Bifidobacterium animalis subsp. lactis DSM10140
- Bifidobacterium pseudolongum subsp. globosum JCM5820
- Bifidobacterium pseudolongum subsp. pseudolongum ATCC25526
- Bifidobacterium thermophilum ATCC25525

As a result, the diameter (mean value) of myotube cells in each sample was as shown in Table 2, and in the system with the culture supernatant of Bifidobacterium, the diameter of the myotube cells was confirmed to be significantly thicker than that of the control, which suggests that muscle mass increased in all strains.

**[Table 2]**

| Table 2: Cell culture test 2 | Myotube diameter (µm) [Mean value ± SD] |
|---|---|
| Control | 18.41±0.46 |
| Bifidobacterium longum subsp. longum ATCC 15707 | 25.39±0.54* |
| Bifidobacterium breve ATCC 15700 | 22.45±0.53* |
| Bifidobacterium bifidum ATCC 29521 | 23.03±0.50* |
| Bifidobacterium adolescentis ATCC 15703 | 23.53±0.59* |
| Bifidobacterium dentium DSM 20436 | 20.96±0.47* |
| Bifidobacterium animalis subsp. lactis DSM 10140 | 21.92±0.47* |
| Bifidobacterium pseudolongum subsp. globosum JCM 5820 | 22.71±0.54* |
| Bifidobacterium pseudolongum subsp. pseudolongum ATCC 25526 | 21.76±0.54* |
| Bifidobacterium thermophilum ATCC 25525 | 21.80±0.49* |
| *P<0.05: VS control | |

### [Test Example 3]

C57BL/6 mice (Charles River, Japan: healthy mice) were used as test animals to evaluate the effect of 4-week continuous ingestion of Bifidobacterium breve FERM BP-11175 on muscle mass increase.

After receiving the test animals at 9 weeks old, F-2 feed (manufactured by Funabashi Pharm) and tap water were freely ingested. After one week of acclimatization, the test animals were divided into 3 groups (n=7/group): a control group (administered 10 mL/kg body weight of tap water); a Bifidobacterium administration group (administered 10 mL/kg body weight of culture supernatant of Bifidobacterium breve FERM BP-11175 prepared in (1) of test example 1); and a leucine administration group (administered 1.5g/kg body weight of leucine), and oral administration was continued for each mouse using an oral probe once a day for 4 weeks. After the final administration, the test animals were fasted for 3 hours, followed by treatment under sevoflurane anesthesia, and plantaris and gastrocnemius was removed to measure the wet weight (body weight ratio) of each tissue. For gastrocnemius, the thin-sectioned tissue was stained with HE, and then the tissue was observed under a microscope.

As a result, the tissue weight (body weight ratio) of each muscle was as shown in Table 3. In the plantaris and gastrocnemius, administration of Bifidobacterium breve FERM BP-11175 was confirmed to increase the tissue weight (body weight ratio) compared to the control. Specifically, administration of Bifidobacterium breve FERM BP-11175 increased the tissue weight in the plantaris by 0.05 mg/g body weight or more compared to the control, and the tissue weight in the gastrocnemius by nearly 0.2 mg/g body weight compared to the control, which confirmed muscle mass increase.

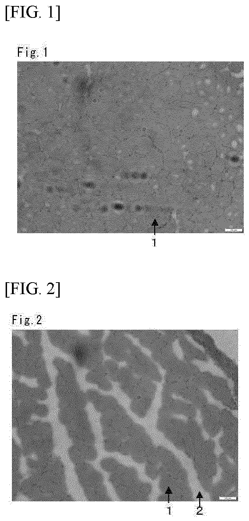

Furthermore, as a result of microscopic observation of the gastrocnemius tissue, as shown in FIG. 1 (Bifidobacterium administration group) and FIG. 2 (control group), in the Bifidobacterium breve FERM BP-11175 administration group, the diameter of the myotube was confirmed to have increased compared with the control group, and the muscle mass was seen to increase.

**[Table 3]**

| Table 3: Mouse test | Plantaris (mg/g body weight) [Mean value ± SE] | Gastrocnemius (mg/g body weight) [Mean value ± SE] |
|---|---|---|
| Control group | 0.645±0.019 | 4.991±0.114 |
| Leucine administration group | 0.706±0.027 | 5.417±0.125 |
| Bifidobacterium administration group | 0.696±0.022 | 5.190±0.153 |

### [Test Example 4]

A randomized, double-blind, placebo-controlled, parallel-group, comparative study of 80 healthy adults with class I obesity (BMI 25 or more and less than 30) was carried out to evaluate the effect of continuous ingestion of Bifidobacterium breve FERM BP-11175 on muscle mass increase.

The 80 subjects were randomly divided into 2 groups, capsules containing Bifidobacterium (Bifidobacterium breve FERM BP-11175) (containing Bifidobacterium equivalent to 20 billion CFU per daily ingestion) or placebo capsules (containing 0.373 g of corn starch (manufactured by Matsutani Chemical Co., Ltd.) per daily ingestion) for each were ingested every day, and muscle mass and skeletal muscle mass were measured after 4 weeks and 8 weeks of ingestion. Measurements were made using an InBody 770 body composition meter (manufactured by Inbody Japan), and the statistically significant difference was analyzed by a t-test. It should be noted that the comparison between groups was evaluated by covariance analysis which used the value before ingestion as a covariate at each evaluation time after ingestion.

As a result, changes in muscle mass were as shown in Table 4, and changes in skeletal muscle mass were as shown in Table 5. Administration of the capsule containing Bifidobacterium (Bifidobacterium breve FERM BP-11175) indicated a significantly higher muscle mass than the placebo group after 8 weeks of ingestion. The change in muscle mass at week 8 was -0.33 kg in the placebo group, which was lower than before ingestion, whereas in the Bifidobacterium-containing capsule administration group (Bifidobacterium administration group), an increase of 0.22 kg was observed. As with muscle mass, skeletal muscle was also significantly higher at week 8 than in the placebo ingestion group. The change at the 8th week was -0.19 kg in the placebo group, whereas in the Bifidobacterium administration group, it increased by 0.19 kg.

**[Table 4]**

| Table 4: Subject test | Week 4 muscle mass (kg) [Mean value ± SE] | Week 8 muscle mass (kg) [Mean value ± SE] |
|---|---|---|
| Placebo group | 54.0±0.17 | 53.8±0.17 |
| Bifidobacterium administration group | 53.9±0.17 | 54.4±0.17* |
| *P<0. 05: VS placebo group | | |

**[Table 5]**

| Table 5: Subject test | Week 4 skeletal muscle mass (kg) [Mean value ± SE] | Week 8 skeletal muscle mass (kg) [Mean value ± SE] |
|---|---|---|
| Placebo group | 32.1±0.10 | 32.0±0.10 |
| Bifidobacterium administration group | 32.1±0.17 | 32.4±0.10* |
| *P<0. 05: VS placebo group | | |

### [Production Example 1]

Bifidobacterium breve FERM BP-11175 was added to 3 mL of MRS liquid medium, anaerobically cultured at 37°C for 16 hours, the culture was concentrated and freeze-dried, and a freeze-dried powder (bacterial cell powder) of the bacteria was obtained. The bacterial powder was mixed uniformly with a whey protein concentrate (WPC) to obtain a composition. 20 g of the composition was dissolved in 200 g of water to obtain a composition for increasing muscle mass. This can also be ingested as a food and beverage for increasing muscle mass, and an effect in increasing muscle mass can be expected.

### [Production Example 2]

Bifidobacterium breve FERM BP-11175 was added to 3 mL of MRS liquid medium, anaerobically cultured at 37°C for 16 hours, the culture was concentrated and freeze-dried, and a freeze-dried powder (bacterial cell powder) of the bacteria was obtained. The bacterial powder was mixed uniformly with dry powder of a milk protein concentrate (MPC480, manufactured by Fontera Co., Ltd., protein content 80% by weight, casein protein:whey protein = about 8:2) to obtain a composition. 20 g of the composition was dissolved in 200 g of water to obtain a composition for increasing muscle mass. This can also be ingested as a food and beverage for increasing muscle mass, and an effect in increasing muscle mass can be expected.

In light of the above, it was confirmed that administration or ingestion of Bifidobacterium and/or a culture thereon promotes muscle hypertrophy by thickening of the myotubes, and that muscle weight increases as well. Therefore, the composition for increasing muscle mass of the present technology may be effectively used for purposes of increasing muscle strengthening effects by training; improvement of frailty or locomotive syndrome; and prevention, improvement, or treatment of muscle diseases, muscle atrophy-related diseases, or symptoms thereof.

### [Description of Reference Numerals]

1 Myotube; 2 Connective tissue

## Claims

1. A composition for increasing muscle mass containing Bifidobacterium and/or a culture of thereof as an active ingredient.

2. The composition according to claim 1, wherein the composition is a pharmaceutical composition.

3. The composition according to claim 1, wherein the composition is a food and beverage composition.

4. A method for increasing muscle mass in which Bifidobacterium and/or a culture of thereof is administered as an active ingredient.

5. A method for improving or treating muscle diseases or symptoms thereof, in which Bifidobacterium and/or a culture of thereof is administered as an active ingredient.

6. Bifidobacterium and/or a culture of thereof for improving or treating muscle diseases or symptoms thereof.

7. Use of Bifidobacterium and/or a culture of thereof for increasing muscle mass.

8. Use of the Bifidobacterium and/or the culture of thereof according to any one of claims 1 to 3 for production of the composition for increasing muscle mass.
